# EUROPEAN PATENT SPECIFICATION

(11) **EP 1 433 492 B1**
(45) Date of publication and mention of the grant of the patent: **18.07.2007**
(21) Application number: 03258043.3
(22) Date of filing: 19.12.2003
(51) Int. Cl.: A61M 1/36

(54) **Irradiation chamber**
Bestrahlungskammer
Chambre d'irradiation

(30) Priority: 20.12.2002 US 434961 P
(43) Date of publication of application: 30.06.2004
(73) Proprietor: THERAKOS, INC., Exton, PA 19341 (US)
(72) Inventor: Gara, Stephen, Collegeville, PA 19426 (US)
(74) Representative: Mercer, Christopher Paul

(56) References cited:
- US-A- 3 628 445
- US-A- 4 574 876
- US-A- 4 578 056
- US-A- 4 737 140
- US-A- 4 876 014
- US-A- 5 263 925

## Description

### FIELD OF THE INVENTION

This invention relates to chamber for holding biological fluid or components thereof such as blood or blood products to facilitate their exposure to electromagnetic radiation such as UV light. This invention can be used in a method of treating cells with radiation thereby inducing apoptosis of the cells such as for the extracorporeal treatment of blood cells, especially leukocytes, with UV radiation.

### BACKGROUND OF THE INVENTION

A number of human diseases are mediated by the overproduction of certain types of leukocytes such as lymphocytes. Excessive or abnormal lymphocyte populations result in numerous adverse effects to patients including the functional impairment of bodily organs, leukocyte mediated autoimmune diseases and leukemia related disorders. Photophoresis therapy has been employed to treat conditions such as CLL, Scleroderma, SLE, Psoriasis, Pemphigus, Psoriatic Arthritis, Atopic dermatitis, ATL, AIDS (ARC), Rheumatoid Arthritis, MS, and organ transplant rejections.

U.S. Pat. Nos. 4,321,919; 4,398,906; 4,428,744; and 4,464,166 to Edelson describe methods for treating blood. These patents describe methods for treating component or components of blood which in turn ameliorate, reduce the severity, or provide relief from the conditions for patients whose blood is treated. In general, the methods comprise treating the blood with a dissolved photoactivatable drug, such as psoralen, which is capable of forming photoadducts with DNA in the presence of U.V. radiation. It is believed that covalent bonding results between the psoralen and the lymphocyte nucleic acid thereby effecting metabolic inhibition of the thusly treated cells. Following extracorporeal radiation, the cells are returned to the patient where they are thought to be cleared by natural processes but at an accelerated pace believed attributable to disruption of membrane integrity, alteration of DNA within the cell, or the like conditions often associated with substantial loss of cellular effectiveness or viability.

In a photophoresis treatment, one or several components of a patient' s blood is exposed to UV radiation in the presence of a photoactivable compound. The phases of a photopheresis treatment comprises collection of the leukocyte-enriched blood or buffy coat, photoactivation and reinfusion with the aid of a patient treatment instrument. Example of an instrument which performs a photopheresis procedure is shown in "The UVAR XTS System: Engineering That Reflects Innovation", dated Mar. 1998. Therakos, Inc. The collection of the buffy coat volume as well as the number of cycles are predetermined by a physician. Assume, that the predetermined volume and cycle conditions are set as follows: 350 mls of plasma, 250 mls of buffy coat, and 5 cycles. In each cycle, the apparatus will collect 250/5 or 50 mls of buffy coat before ending the cycle and thereupon emptying the centrifuge bowl and returning all nonleukocyte fluids, predominantly erythrocytes and perhaps excess plasma, to the patient. Prior to the collection of the 50 mls, plasma will emerge from the centrifuge and will be collected either until the full 350 mls are collected or, until the buffy coat emerges.

More specifically, the instrument collects and separates blood on a continuous basis as it is withdrawn from the patient and returns untreated portions to the patient while concurrently irradiating the buffy coat in the irradiation chamber with UV light. The irradiation, preferably UV light, photoactivates the photoactivatable agent in contact with the desired blood portion while the agent and the cells (or other patient fluid) is contained within the flat plate irradiation chamber. Following photoactivation, the treated cells will be returned to the patient utilizing a drip chamber gravity feed infusion line incorporated in a photopherisis blood tubing set. The photopheresis blood tubing set has multiple lines used for collecting, photoactivating and reinfusing the leukocyte-enriched blood. These lines are the patient/heparin line, the collection/return line, the bowl outlet line and the photoactivation line. ,

The instrument also controls blood and recirculation pump speed/direction and also supplies power to the centrifuge. A microprocessor and discrete logic circuits monitor operating parameters throughout treatment and display instrument status and conditions. Microprocessor controls assist the operator in the various stages of the photopheresis procedure.

The irradiation chamber has a thin sterile fluid pathway constructed of UVA-transparent acrylic. The irradiation chamber's design allows it to be inserted between the two banks of UVA lamps for photoactivation. Suitable sources for UV lamps include the Sylvania FR15"T8/350BL/HO/180 degree with 2011 phosphorus bulb which is in the so-called fluorescent tube form.

Photoactivable compounds that can be used with the present invention include, but are not limited to, primaryamino-pyrone-linked and benzene-linked psoralens disclosed in U.S. Patent No. 6,455,286. Compound 8-methoxy psoralen is most preferred among photoactivatable compounds in the psoralen class.

There are many patents and publications that disclose containers/chambers for treatment of fluid, including blood products, by irradiation (e.g. U.S. Patent Nos. 3,628,445; 4,708,715; 4,737,140; D298,279; 4,866,282; 4,876,014; 4,897,789; 4,915,683; 5,039,483; 5,304,113; 5,290,221; 5,868,695; 5,951,509; 6,133,566; 6,312,593 and US2001/0024623).

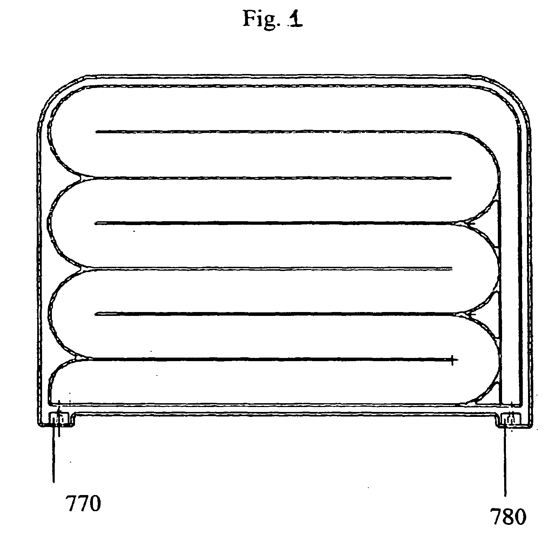

One device, is shown in publications WO 98/22165, WO 98/22163 and in a brochure dated March 1998 by Therakos, Inc., "The UVAR XTS System: Engineering That Reflects Innovation." These publications show an irradiation chamber having seven channels where blood products pass through while being irradiated with UV light. The irradiation chamber, also known as the PHOTOCEPTOR^{®} Photoactivation Chamber, is made from two differently shaped plates. One side of the irradiation chamber has both input and output ports protruding above the surface of the plate (Fig. 1 shows input and output ports 770 and 780 on opposite side of front plate) while the other side is essentially flat (Fig. 1, front plate shown). Furthermore, one plate has partitions extending from its surface, which are then sealed to recesses of the other plate to form a serpentine pathway.

The current manufacturing process to produce the PHOTOCEPTOR^{®} Photoactivation Chamber results in a number of rejected products. This is due to uneven application of RF energy between the two different plates in the manufacturing process: one plate has partitions extending perpendicular from its surface and one has recesses on its surface to receive the partitions. In addition to the difficulty in the production of this irradiation chamber, the manufacturing process requires two injection mold tools for the two plates.

Displacement of a desired buffy coat from the PHOTOCEPTOR^{®} Photoactivation Chamber requires introduction of another fluid such as plasma or saline because the design of the irradiation chamber does not effectively utilize gravity to transfer blood components. The volume of such fluid usually exceeds the volume of the irradiation chamber due to a wash volume required for residual buffy. In a typical photopherisis treatment for normal adult patients requiring several cycles of separation, irradiation, and reinfusion of blood, additional volumes of fluid may be returned to the patient. However in a small patient (such as children) or a patient whose vascular system is easily overloaded with fluids, the extra volume of fluid provided in extracorporeal photopherisis potentially presents a problem.

The design of the irradiation chamber of the present invention allows for gravity-assisted transferring of a desired fluid. Gravity-assisted transferring of fluid results in greater efficiency in fluid transfer management and allows for reducing the total volume being processed in an extracorporeal photopheresis treatment. For example, if the desired fluid is bufffy coat, then displacement of the buffy coat is accomplished by a displacing plasma and/or saline with the aid of gravity. A washing volume may be needed for residual buffy coat, however this washing volume is small relative to that needed for the PHOTOCEPTOR^{®} Photoactivation Chamber. This ability to have a reduced processed volume is desirable for small patients or those with compromised vascular systems.

The present invention also allows for efficient manufacturing of irradiation chamber due to fewer mold tools needed because the two plates forming the irradiation chamber are identical. Only one mold is required for the production of the irradiation of the present invention. Furthermore, the chambers giving rise to the serpentine pathway of the irradiation chamber are formed by RF welding of partitions extending perpendicular from a plate's surface. RF welding of partitions of the present invention to form a serpentine pathway provides fewer rejected product due to even application of RF energy to partitions of plates.

It is a further related object of this invention to provide an irradiation chamber that can be used with a continuous on-line patient treatment system wherein collection, separation, and cell treatment occur simultaneously.

### BRIEF DESCRIPTION OF THE DRAWINGS

These and still other objects of the invention will become apparent upon study of the accompanying drawings:
FIG. 1 shows a front view of a PHOTOCEPTOR^{®} Photoactivation Chamber.
FIG. 2 shows a front view of the claimed irradiation chamber.
FIG. 3 shows a side longitudinal view of the claimed irradiation chamber.
FIG. 4 shows a side transverse view of the claimed irradiation chamber.

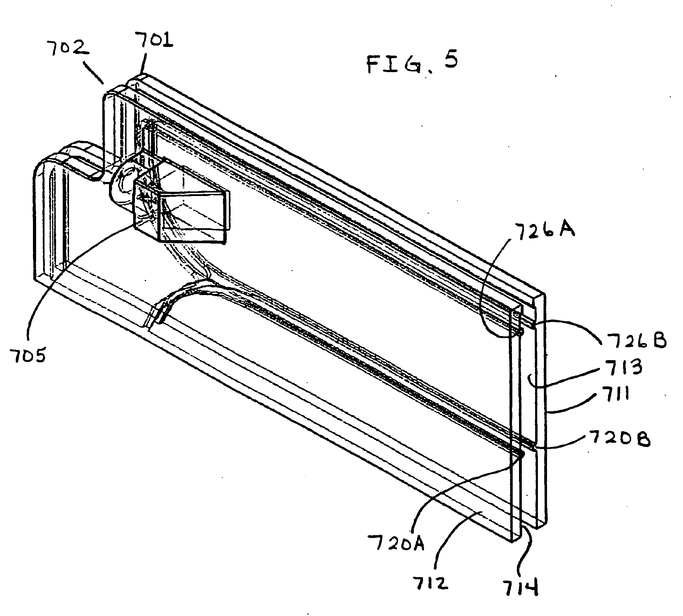

FIG. 5 shows a cut-away view of a section of the first plate and the second plate prior to being joined together.
FIG. 6 shows a cut-away dimensional end view of an irradiation chamber.
FIG. 7 shows an irradiation chamber in a UVA light assembly.

### SUMMARY OF THE INVENTION

The present invention provides for an irradiation chamber comprising: a rigid first plate having a first surface and a second surface having a raised boundary surrounding a plurality of raised partitions; a rigid second plate having a first surface and a second surface having a raised boundary surrounding a plurality of raised partitions, wherein the second surface of said rigid first plate is contacted with second surface of said rigid second plate thereby forming a chamber; said chamber, defined by the raised boundary surrounding the plurality of raised partitions which extend from said second surface of said first plate and said second surface of said second plate, said chamber having a first port and a second port, wherein a plurality of channels are formed by said partition and are in fluid communication with the first port and second port.

### DETAILED DESCRIPTION

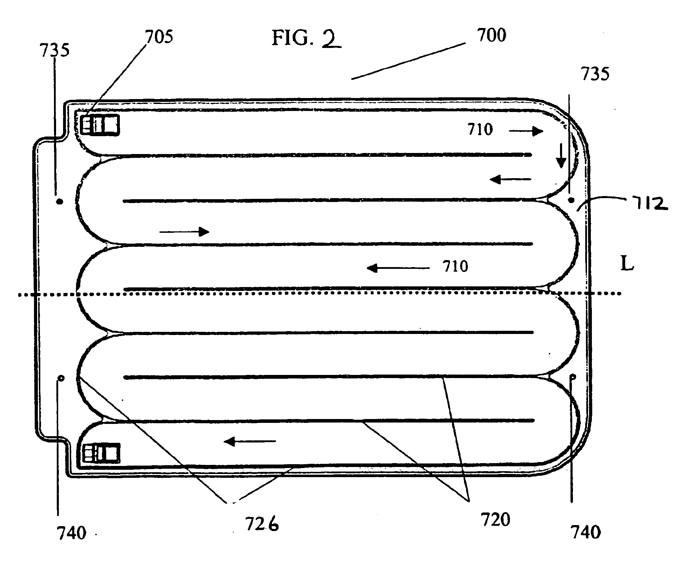

Irradiation chamber 700 (Fig. 2) is formed by joining two plates, a front and a back plate having a thickness of preferably about 0.06 in. to about 0.2 in., which are preferably comprised of a material ideally transparent to the wavelength of electromagnetic radiation. In the case of ultraviolet A radiation, polycarbonate has been found most preferred although other materials such as acrylic may be employed. Similarly, many known methods of bonding may be employed and need not be expanded on here.

The first plate 702 has a first surface 712 and a second surface 714. In a preferred embodiment the first plate 702 has a first port 705 on a first surface 712, in fluid communications with the second surface 714. The second surface 714 of the first plate 702 has a raised boundary 726A defining an enclosure. The boundary 726A preferably extends substantially perpendicular from the second surface 714 (i.e. about 80-100 degrees). Extending from the second surface 714 (preferably substantially perpendicularly) are raised partitions 720A. The boundary 726A surrounds the partitions 720A. One end of each partition 720A extends and contacts the boundary 726A.

The second plate 701 has a first surface 711 and a second surface 713. In a preferred embodiment the second plate 701 preferably has a second port 730 on a first surface 711, in fluid communications with the second surface 713. The second surface 713 of the back plate 701 has a raised boundary 726B defining an enclosure. The boundary 726B preferably extends substantially perpendicular from the second surface 713 (i.e. about 80-100 degrees). Extending from the second surface 713 (preferably substantially perpendicular) are raised partitions (720B). The boundary 726B surrounds the partitions 720B. One end of each partition 720A extends and contacts one side of boundary (726B).

The joining of the second surfaces of the first and second plates results in a fluid tight junction between boundaries 726A and 726B thereby forming boundary 726. Partitions 720A and 720B are also joined forming a fluid tight junction thereby forming partition 720. The boundary 726 forms an irradiation chamber 700 and together with the partitions 720 provides a pathway 710 having channels 715 for conducting fluid. The pathway maybe serpentine, zig-zag, or dove-tailed. Currently preferred is a serpentine pathway.

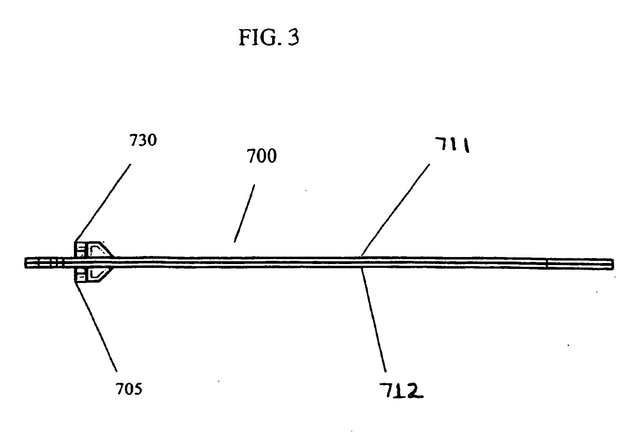

With reference to FIG. 2 and 3, irradiation chamber 700 comprises a serpentine pathway 710 for conducting patient fluid from inlet port 705 to outlet port 730, i.e., the serpentine pathway 710 is in fluid communication with inlet port 705 of front plate 702 and outlet port 730 of back plate 701. Self-shielding effects of the cells is reduced while the cells are photoactivated by irradiation impinging upon both sides of irradiation chamber 700.

Figure 2 shows pin 740 and recess 735 which align the two plates of irradiation chamber prior to being joined together in a sealing arrangement by RF welding, heat impulse welding, solvent welding or adhesive bonding. Joining of the plates by adhesive bonding and RF welding is more preferred. Joining of the front and back plates by RF welding is most preferred as the design of the raised partitions 720 and perimeter 725 minimizes flashing and allows for even application of RF energy. Locations of pin 740 and recess 735 may be inside serpentine pathway 710 or outside of serpentine pathway 710 (as shown in Fig.2). Figure 2 also shows a view of an irradiation chamber with axis L. Rotation of chamber 180 degree about axis L gives the original configuration of the irradiation chamber. The irradiation chamber of the present invention has C₂ symmetry about axis L.

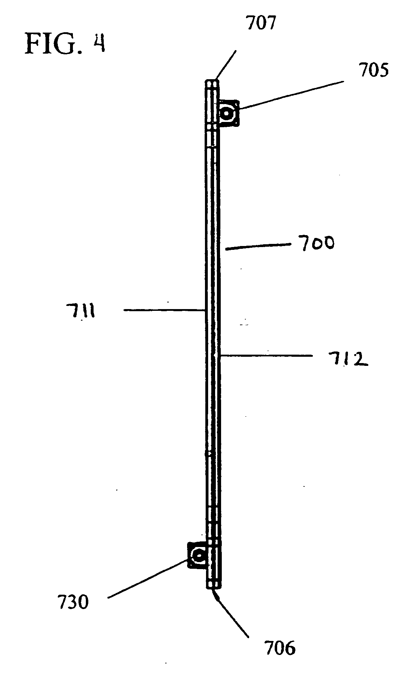

Referring to FIG. 2, 4, and 7, the leukocyte enriched blood, plasma, and priming solution are delivered through inlet port 705 of front plate 702 of irradiation chamber 700 into channel 715. The channel 715 in the irradiation chamber 700 is relatively "thin" (e.g. on the order of approximately 0.04" as distance between two plates) in order to present large surface area of leukocyte rich blood to irradiation and reduce the self-shielding effects encountered with lower surface area/volume ratios. The cross section shape of channel 715 is substantially rectangular (e.g. rectangular, rhomboidal or trapezoidal) which has as its long side the distance between partition 720 and the distance between the plates as its short side. The shape of the cross section is designed for optimal irradiation of cells passing through channel 715. While a serpentine pathway 710 is preferred in order to avoid or minimize stagnant areas of flow, other arrangements are contemplated.

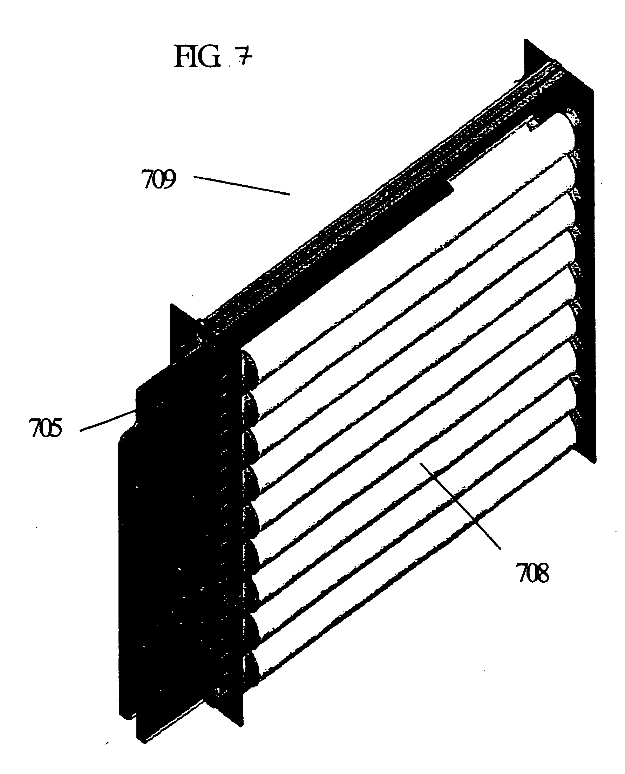

The irradiation chamber 700 allows efficient activation of photoactivatable agents by irradiation from a light array assembly such as the PHOTOSETTE^{®}'s two banks of UVA lamps (708) for activation (Figure 7). The irradiation chamber and UVA light assembly (709) is designed to be used in a setting where edge 706 is oriented downward and edge 707 points upward. In this orientation, fluids entering input port 705 can exit from outlet port 730 with the aid of gravity. In the most preferred embodiment, irradiation of both sides of the irradiation chamber takes place concurrently while still permitting facile removal of the chamber.

The irradiation chamber's fluid pathway loops to form two or more channels in which the leukocyte-enriched blood is circulated during photoactivation by UVA light. Preferably, irradiation chamber has between 4 to 12 channels. More preferably, the irradiation chamber has 6 to 8 channels. Most preferably, the irradiation chamber has 8 channels.

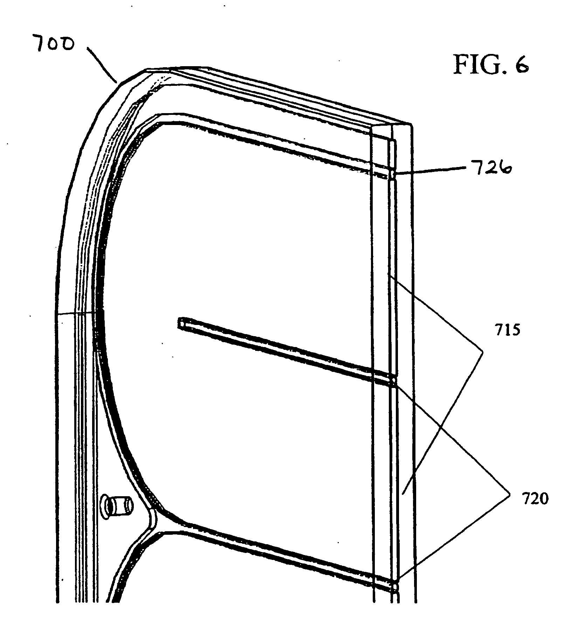

Figure 6 shows cut-away views of the irradiation chamber. The channels 715 of serpentine pathway 710 are formed by the joining of raised partition 720 and perimeter 726 of the plates.

The irradiation chamber of the present invention can be made from a biocompatible material and can be sterilized by known methods such as heating, radiation exposure or treatment with ethylene oxide (ETO).

## Claims

1. An irradiation chamber comprising:
a rigid first plate having a first surface and a second surface said second surface having a raised boundary surrounding a plurality of raised partitions;
a rigid second plate having a first surface and a second surface, said second surface having a raised boundary surrounding a plurality of raised partitions;
wherein the second surface of said rigid first plate is contacted with second surface of said rigid second plate thereby forming a chamber; said chamber, defined by the raised boundary surrounding the plurality of raised partitions which extend from said second surface of said first plate and said second surface of said second plate, said chamber having a first port and a second port, wherein a plurality of channels are formed by said partition providing fluid communication with the first port and second port
**characterised in that** said rigid first plate is identical to said rigid second plate.

2. The irradiation chamber of Claim 1 wherein said partitions are essentially evenly spaced apart from each other.

3. The irradiation chamber of Claim 1 or Claim 2 wherein the number of partitions ranges from three to eleven.

4. The irradiation chamber of Claim 3 wherein the number of partitions is seven.

5. The irradiation chamber of any one of Claims 1 to 4 wherein the channels form a serpentine fluid pathway providing fluid communications between said first and second port.

6. The irradiation chamber of Claim 5 wherein the first port is on the first surface of the first plate.

7. The irradiation chamber of Claim 6 wherein the second port is on the first surface of the second plate.

8. The irradiation chamber of any one of Claims 1 to 7, wherein the channel has a thickness of about 0.04 inches.

9. The irradiation chamber of any one of Claims 1 to 8, wherein the rigid first plate and second plate are made of a material that does not substantially absorb UV radiation having wavelength in the range of 180 to 420 nm.

10. The irradiation chamber of claim 9, wherein the rigid first plate and second plate are made of polycarbonate or acrylic.

## Patentansprüche

1. Bestrahlungskammer, mit:
einer starren ersten Platte, die eine erste Oberfläche und eine zweite Oberfläche hat, wobei die zweite Oberfläche eine erhöhte Begrenzung hat, die eine Vielzahl erhöhter Trennwände umgibt;
einer starren zweiten Platte, die eine erste Oberfläche und eine zweite Oberfläche hat, wobei die zweite Oberfläche eine erhöhte Begrenzung hat, die eine Vielzahl erhöhter Trennwände umgibt;
wobei die zweite Oberfläche der starren ersten Platte im Kontakt mit der zweiten Oberfläche der starren zweiten Platte ist, so daß eine Kammer gebildet wird, wobei die Kammer durch die erhöhte Begrenzung definiert ist, die die Vielzahl der Trennwände umgibt, welche sich von der zweiten Oberfläche der ersten Platte und von der zweiten Oberfläche der zweiten Platte erstrecken, wobei die Kammer einen ersten Port und einen zweiten Port hat, wobei eine Vielzahl von Kanälen durch die Trennwände gebildet wird, welche eine Fluidverbindung mit dem ersten Port und dem zweiten Port herstellt,
**dadurch gekennzeichnet, daß** die erste Platte mit der zweiten Platte baugleich ist.

2. Bestrahlungskammer nach Anspruch 1, bei der die Trennwände voneinander im wesentlichen gleich beabstandet sind.

3. Bestrahlungskammer nach Anspruch 1 oder Anspruch 2, bei der die Anzahl der Trennwände im Bereich von drei bis elf liegt.

4. Bestrahlungskammer nach Anspruch 3, bei der die Anzahl der Trennwände sieben ist.

5. Bestrahlungskammer nach einem der Ansprüche 1 bis 4, bei der die Kanäle einen gewundenen Fluidweg bilden, welcher eine Fluidverbindung zwischen dem ersten Port und dem zweiten Port herstellt.

6. Bestrahlungskammer nach Anspruch 5, bei der der erste Port auf der ersten Oberfläche der ersten Platte ist.

7. Bestrahlungskammer nach Anspruch 6, bei der der zweite Port auf der ersten Oberfläche der zweiten Platte ist.

8. Bestrahlungskammer nach einem der Ansprüche 1 bis 7, bei der der Kanal eine Dicke von ungefähr 0.04 Zoll hat.

9. Bestrahlungskammer nach einem der Ansprüche 1 bis 8, bei der die starre erste Platte und zweite Platte aus einem Material hergestellt sind, welches UV-Strahlung mit einer Wellenlänge in dem Bereich von 180 bis 420 nm nicht wesentlich absorbiert.

10. Bestrahlungskammer nach Anspruch 9, bei der die starre erste Platte und zweite Platte aus Polycarbonat oder Acryl hergestellt sind.

## Revendications

1. Chambre d'irradiation comprenant :
une première plaque rigide présentant une première surface et une seconde surface, ladite seconde surface comportant une limite surélevée entourant une pluralité de cloisons surélevées ;
une seconde plaque rigide présentant une première surface et une seconde surface, ladite seconde surface comportant une limite surélevée entourant une pluralité de cloisons surélevées ;
dans laquelle la seconde surface de ladite première plaque rigide est en contact avec la seconde surface de ladite seconde plaque rigide, formant ainsi une chambre ; ladite chambre, définie par la limite surélevée entourant la pluralité de cloisons surélevées qui s'étendent depuis la seconde surface de ladite première plaque et ladite seconde surface de ladite seconde plaque, ladite chambre comportant un premier orifice et un second orifice, une pluralité de canaux étant formés par ladite cloison permettant une communication de fluide avec le premier orifice et le second orifice,
**caractérisée en ce que** ladite première plaque rigide est identique à ladite seconde plaque rigide.

2. Chambre d'irradiation selon la revendication 1, dans laquelle lesdites cloisons sont espacées les unes des autres de manière essentiellement égale.

3. Chambre d'irradiation selon la revendication 1 ou la revendication 2, dans laquelle le nombre de cloisons est compris entre trois et onze.

4. Chambre d'irradiation selon la revendication 3, dans laquelle le nombre de cloisons est de sept.

5. Chambre d'irradiation selon l'une quelconque des revendications 1 à 4, dans laquelle les canaux forment un passage de fluide sinueux permettant des communications de fluide entre lesdits premier et second orifices.

6. Chambre d'irradiation selon la revendication 5, dans laquelle le premier orifice se trouve sur la première surface de la première plaque.

7. Chambre d'irradiation selon la revendication 6, dans laquelle le second orifice se trouve sur la première surface de la seconde plaque.

8. Chambre d'irradiation selon l'une quelconque des revendications 1 à 7, dans laquelle le canal présente une épaisseur d'environ 0,04 pouce.

9. Chambre d'irradiation selon l'une quelconque des revendications 1 à 8, dans laquelle la première plaque rigide et la seconde plaque rigide sont composées d'un matériau qui n'absorbe pratiquement pas les rayons UV ayant une longueur d'onde comprise dans la plage de 180 à 450 mm.

10. Chambre d'irradiation selon la revendication 9, dans laquelle la première plaque rigide et la seconde plaque rigide sont composées de polycarbonate ou d'acrylique.
